# EUROPÄISCHE PATENTSCHRIFT

(11) **EP 1 491 505 B1**
(45) Veröffentlichungstag und Bekanntmachung des Hinweises auf die Patenterteilung: **03.04.2013**
(21) Anmeldenummer: 04013312.6
(22) Anmeldetag: 05.06.2004
(51) Int. Cl.: C02F 1/00, G01N 33/18, G01N 33/50, C12Q 1/00

(54) **Verfahren zur Messung und zur Kontrolle der Biofilmbildung in einem Wassersystem**
Method of measuring and controlling the formation of biofilms in a watersystem
Procédé de mesure et control de la formation de biofilms dans un système d'eau

(30) Priorität: 13.06.2003 DE 10326586
(43) Veröffentlichungstag der Anmeldung: 29.12.2004
(73) Patentinhaber: BK Giulini GmbH, 67065 Ludwigshafen (DE)
(72) Erfinder: Breves, Roland, 40822 Mettmann (DE); Janssen, Frank, 40589 Düsseldorf (DE); Hater, Wolfgang, 41564 Kaarst (DE); Gomes Machado, Paulo Jorge, 50670 Köln (DE)

(56) Entgegenhaltungen:
- PEDERSEN K: "METHOD FOR STUDYING MICROBIAL BIO FILMS IN FLOWING WATER SYSTEMS" APPLIED AND ENVIRONMENTAL MICROBIOLOGY, Bd. 43, Nr. 1, 1982, Seiten 6-13, XP009038610 ISSN: 0099-2240
- PEDERSEN K: "FACTORS REGULATING MICROBIAL BIO FILM DEVELOPMENT IN A SYSTEM WITH SLOWLY FLOWING SEA WATER" APPLIED AND ENVIRONMENTAL MICROBIOLOGY, Bd. 44, Nr. 5, 1982, Seiten 1196-1204, XP009038611 ISSN: 0099-2240
- O'TOOLE G A ET AL: "Genetic approaches to study of biofilms." METHODS IN ENZYMOLOGY. 1999, Bd. 310, 1999, Seiten 91-109, XP009038573 ISSN: 0076-6879
- DJORDJEVIC D ET AL: "Microtiter plate assay for assessment of Listeria monocytogenes biofilm formation" APPLIED AND ENVIRONMENTAL MICROBIOLOGY, Bd. 68, Nr. 6, Juni 2002 (2002-06), Seiten 2950-2958, XP002302199 ISSN: 0099-2240
- HEILMANN CHRISTINE ET AL: "Characterization of Tn917 insertion mutants of Staphylococcus epidermidis affected in biofilm formation" INFECTION AND IMMUNITY, Bd. 64, Nr. 1, 1996, Seiten 277-282, XP002302200 ISSN: 0019-9567
- LI XIAOGANG ET AL: "Quantitative variation of biofilms among strains in natural populations of Candida albicans." MICROBIOLOGY (READING), Bd. 149, Nr. 2, Februar 2003 (2003-02), Seiten 353-362, XP002302201 ISSN: 1350-0872

## Beschreibung

Die vorliegende Erfindung betrifft ein Verfahren zur Messung und zur Kontrolle der Bildung von sogenannten "Biofilmen" in einem Wassersystem, insbesondere in einem offenen oder geschlossenen Kühl- oder Prozeßwasserkreislauf. Unter "Messung" wird dabei verstanden, das Ausmaß der tatsächlichen Biofilmbildung zumindest mit einer derartigen Genauigkeit zu bestimmen, daß hieraus Folgerungen für eine erforderliche Wasserbehandlung abgeleitet werden können. "Kontrolle" heißt, durch eine entsprechende Zugabe von geeigneten Wasserbehandlungsprodukten die Biofilmbildung in tolerierbaren Grenzen zu halten.

Biofilme sind Ablagerungen auf Oberflächen, die von Mikroorganismen, insbesondere von Bakterien, durch Ausscheidung von Stoffwechselprodukten gebildet werden. Der Biofilm besteht also aus den ausgeschiedenen filmbildenden Produkten und den in dieser Matrix lebenden oder bereits abgestorbenen Mikroorganismen. Die Biofilm-Matrix schützt die Mikroorganismen vor für sie toxischen Stoffen wie beispielsweise Bioziden. Daher sind Mikroorganismen in einem Biofilm deutlich schwieriger zu bekämpfen als solche, die frei in der Wasserphase umhertreiben. Biofilme haften hartnäckig auf der Unterlage und sind durch chemische Mittel nur schwer zu entfernen. Die Bildung von Biofilmen stellt daher ein allgemeines und schwierig zu beherrschendes Problem in allen Wassersystemen dar, in denen Mikroorganismen wie Bakterien überhaupt lebensfähig sind. Beispielsweise treten derartige Probleme in Trinkwasserleitungen, Kühlwasserkreisläufen oder anderen technischen Prozeßflüssigkeiten, z. B. in Kraftwerken, Stahl- und Walzwerken, Papierfabriken usw. auf. Neben einer mechanischen Behinderung des Strömungsverhaltens bis hin zur Verstopfung von Rohren und Ventilen bewirken die mikrobiell gebildeten Beläge auch eine Reduktion des Wärmeübergangs auf Wärmeaustauscheroberflächen.

Ein bereits gebildeter Biofilm ist nur schwierig wieder aufzulösen. Daher versucht man, durch Vorbeugungsmaßnahmen eine Bildung von Biofilmen zu verhindern. Hierzu werden dem Wassersystem üblicherweise Biozide zugegeben. Um sowohl eine wirtschaftlich und ggf. auch ökologisch nachteilige Überdosierung als auch eine Unterdosierung zu vermeiden, ist es wünschenswert, möglichst frühzeitig eine beginnende Biofilmbildung zu erkennen und zumindest halbquantitativ zu beurteilen.

Wegen ihrer hohen wirtschaftlichen, technischen und gesundheitlichen Bedeutung wurden Biofilme umfangreich untersucht. Einen Überblick über die Biofilmbildung in Wassersystemen gibt H.-C. Flemming: "Biofouling in water systems - cases, causes, and countermeasures", App. Microbiol. Biotechnol. 59, SS. 629 - 640 (2002). Dort wird mitgeteilt, daß die meisten Mirkoorganismen Biofilme bilden können. In ihnen sind die Mikroorganismen in einer Matrix mikrobiologischen Ursprungs eingebettet, die aus extracellularen polymeren Substanzen besteht. Diese umfassen hauptsächlich Polysaccharide und Proteine, die eine Hydrogel-artige Matrix bilden. Als Behandlungsstrategie für eine Biofilmbildung wird empfohlen, nicht alle Organismen in dem Wassersystem abzutöten, sondern sie unterhalb einer harmlosen Grenzpopulation zu halten. Hierzu muß die Biofilmbildung überwacht und durch geeignete Mittel bekämpft werden. Eine einfache Wasseranalyse reicht hierfür nicht aus. Vielmehr ist es erforderlich, Prüfkörper in das Wassersystem einzubringen und auf Biofilmbildung zu untersuchen.

In G.A. O'Toole, L.A. Pratt, P.I. Watnick, D.K. Newman, V.B. Weaver, R. Kolter: "Genetic Approaches to Study of Biofilms", in: "Methods in Enzymology, Vol. 310 "Biofilms", herausgegeben von R.J. Doyle, Academic Press 1999, SS. 91 - 109wird das Wachstum von Biofilmen unter Laborbedingungen untersucht. Die Biofilmbildung wird quantitativ durch Anfärben mit Safranin und durch Messung der Lichtabsorption bei 490 nm verfolgt. Eine ähnliche Anfärbemethode verwendet auch die Forschungsarbeit Ch. Heilmann, Ch. Gerke, F. Perdreau-Remington, F. Götz: "Characterization of Tn917 Insertion Mutants of Staphylococcus epidermidis Affected in Biofilm Formation", Infection and Immunity, Januar 1996, SS. 277 - 282.

In der Patentliteratur sind ebenfalls einige Methoden beschrieben, wie die Biofilmbildung in einem Wassersystem gemessen werden kann. Beispiele hierfür sind: WO 02/10434, US 5 049 492, WO 95/27039 und US 5 641 642. In keinem dieser Verfahren erfolgt eine quantitative Bestimmung der Menge des gebildeten Biofilms mittels Anfärbemethoden. Häufig wird lediglich die Anzahl der Keime pro Flächeneinheit bestimmt. Dies geschieht auch in einem Verfahren, das von der Firma PS Biofilm Technology kommerziell angeboten wird (Verfahrensbeschreibung von PS Biofilm Technology: "Part I: Biofilm Development Assemblies and Multiple Monitoring System", ohne Jahresangabe).

Gemäß WO 01/49876 kann man planktonische und sessile mikrobiologische Populationen in einem industriellen Wassersystem kontrollieren, indem man dem Wassersystem einen Fluoreszenzfarbstoff zugibt, der mit den Mikroorganismen reagieren kann. Durch Messung der Fluoreszenz von reagiertem und nichtreagiertem Farbstoff bzw. von reagiertem Farbstoff und einer nichtreaktiven Vergleichssubstanz können Aussagen über das Ausmaß des biologischen Wachstums gemacht und hierüber die Biozidzugabe gesteuert werden. Hierbei wird jedoch generell die mikrobiologische Aktivität und nicht speziell die Biofilmbildung erfasst.

Die vorliegende Erfindung stellt sich die Aufgabe, ein vereinfachtes Verfahren zur Messung und zur Kontrolle der Biofilmbildung in einem Wassersystem zur Verfügung zu stellen. Dieses Verfahren soll auch von nicht akademisch ausgebildetem Personal durchgeführt werden können. Vorzugsweise sollen hierbei Einrichtungen benutzt werden können, die in dem untersuchten Wassersystem wie beispielsweise Kühlkreisläufen üblicherweise vorhanden sind. Solche Einrichtungen sind beispielsweise Einbauten zum Einbringen von Coupons zur Korrosionskontrolle.

Gegenstand der vorliegenden Erfindung ist ein Verfahren zur Messung der Biofilmbildung in einem Wassersystem, wobei man in das Wassersystem selbst oder in eine Zweigleitung einen oder mehrere Prüfkörper einbringt und nach einer vorgewählten Zeitdauer entnimmt, dadurch gekennzeichnet, dass man
a) fakultativ, d.h. nicht notwendigerweise, den Prüfkörper mit einer ersten wässrigen Lösung abspült,
b) anschließend den Biofilm auf dem Prüfkörper mit einer Farbstofflösung einfärbt,
c) anschließend den Prüfkörper mit einer zweiten wässrigen Lösung abspült, deren Zusammensetzung der ersten wässrigen Lösung entsprechen oder von dieser verschieden sein kann, und anschließend die Intensität der Färbung des Biofilms ermittelt, indem man
d) den im Biofilm gebundenen Farbstoff von dem Prüfkörper mit einem Lösungsmittel ablöst, wobei man eine Farbstofflösung erhält, deren Farbintensität man durch Vergleich mit Vergleichs-Farbstofflösungen beurteilt,
e) und dann, wenn die im Teilschritt d) ermittelte Intensität der Färbung des Biofilms einen vorgegebenen Schwellenwert übersteigt, dem Wassersystem mindestens ein Biozid und/oder einen Biodispergator zugibt.

Im erfindungsgemäßen Verfahren wird also nicht die Anzahl lebender Keime im Biofilm ermittelt, sondern das Ausmaß der Biofilmbildung selbst. Diese beiden Größen korrelieren nicht zwangsläufig miteinander, da die pro Keim gebildete Menge an Biofilm-Matrix von den Lebensbedingungen am Ort des Biofilms abhängt.

Für Vergleichszwecke kann man die flächenbezogene Trockenmasse des Biofilms auf dem Prüfkörper dadurch feststellen, daß man die im Teilschritt d) ermittelte Intensität der Färbung des Biofilms mit der auf die selbe Weise ermittelten Intensität der Färbung von Biofilmen mit bekannten flächenbezogenen Trockenmassen vergleicht. Hierdurch kann die Biofilmbildung an unterschiedlichen Orten eines Wassersystems oder in unterschiedlichen Wassersystemen verglichen werden.

Ist in dem Wassersystem eine Einrichtung zum Einbringen von Coupons zur Korrosionskontrolle vorgesehen, so kann diese Einrichtung vorteilhafterweise zumindest teilweise für das erfindungsgemäße Verfahren verwendet werden. Eine derartige Einrichtung ist beispielsweise in dem ASTM-Standard D 2688-94 beschrieben (ASTM = "American Society for Testing and Materials", 1916 Race Street, Philadelphia, Pa 19103, USA). Anstelle der Testcoupons zur Korrosionsmessung bringt man die Prüfcoupons für die Biofilmbildung ein. Hierdurch ist es nicht erforderlich, zur Ausführung des erfindungsgemäßen Verfahrens spezielle Installationen an dem Wassersystem vorzunehmen.

Der Prüfkörper ist vorzugsweise flächenförmig ausgebildet, so daß der Biofilm auf einer Fläche aufwachsen kann. "Flächenförmig ausgebildet" heißt, daß der Prüfkörper ein hohes Verhältnis von Oberfläche zu Volumen aufweist, d.h. zwei Längendimensionen sind mindestens 5 mal so groß wie die dritte. Vorzugsweise ist die Fläche eben, kann aber auch zum erleichterten Einbau gekrümmt oder zu einem Zylinder oder Rohr gebogen sein. Vorzugsweise besteht der Prüfkörper aus einem Material, das in dem Wassersystem nicht korrodiert, so daß das Wachstum des Biofilms nicht durch chemische Prozesse an der Oberfläche des Prüfkörpers beeinflusst wird. Beispielsweise eignen sich Prüfkörper aus Edelstahl, Glas, Kunststoff und Keramik.

Man lässt den Prüfkörper für eine vorgewählte Zeitdauer in dem Wassersystem und entnimmt und untersucht ihn nach Ablauf dieser vorgewählten Zeitdauer. Die Zeitdauer hängt von der Tendenz des Wassersystems zur Biofilmbildung ab und kann empirisch optimiert werden. Eine Zeitdauer im Bereich von etwa 5 Tagen bis etwa 5 Monaten sollte generell geeignet sein. Vorzugsweise wählt man eine Zeitdauer im Bereich von etwa 2 Wochen bis etwa 2 Monaten. Dies stellt in der Regel einen vernünftigen Kompromiss zwischen Messdauer und Aussagekraft der Messung dar.

Vorzugsweise bringt man den Prüfkörper so in das Wassersystem oder eine Zweigleitung ein, daß nach dem Einbringen kein Licht auf den Prüfkörper fällt. Dies heißt, die Umhüllung des Wassersystems, beispielsweise die Rohrleitung, soll an der Stelle des Prüfkörpers undurchsichtig sein. Hierdurch verhindert man eine Verfälschung der Messung durch das Wachstum photosynthetisch aktiver Organismen wie beispielsweise Algen.

Zum Anfärben des Biofilms nach der Entnahme des Prüfkörpers wird dieser erwünschtenfalls im fakultativen Schritt a) mit einer ersten wässrigen Lösung abgespült. Dabei ist es empfehlenswert, Bedingungen einzuhalten, die den Biofilm auf dem Prüfkörper möglichst wenig verändern und in seinem letzten Zustand "konservieren". Dies wird beispielsweise dadurch erreicht, daß man eine wässrige Lösung verwendet, die auf einen pH-Wert im Bereich von 7,0 bis 8,0 abgepuffert ist und die mindestens eine Substanz enthält, die das Wachstum von Bakterien hemmt. Für das Abpuffern ist beispielsweise ein Dihydrogenphosphat/Hydrogenphosphat-Puffer, aber auch jedes andere Puffersystem für diesen pH-Bereich geeignet. Bei geeignetem pH-Wert des zu beprobenden Flüssigkeitssystems kann auf Puffersubstanzen auch ganz verzichtet werden. Verwendet man beispielsweise einen K/Na-Phosphat-Puffer, setzt man diesen vorzugsweise mit einer Konzentration im Bereich von 1 bis 150 millimolar, insbesondere von 2 bis 20 millimolar ein. Weiterhin kann die wässrige Lösung bis zu 9 g/l NaCl enthalten. Als Substanz, die das Wachstum von Bakterien hemmt, können Azidionen, beispielsweise in Form von Natriumazid, gewählt werden. Die Konzentration von Natriumazid liegt vorzugsweise im Bereich von 0,005 bis 0,2 Gew.-%, insbesondere bei 0,02 Gew.-%. Eine geeignete Lösung enthält beispielsweise 0,21 g/l KH₂PO₄, 9,00 g/l NaCl, 0,726 g/l Na₂HPO₄ x 7H₂O sowie 0,02 Gew.-% Natriumazid. Sie hat einen pH-Wert von 7,4.

Im Teilschritt b) färbt man den Biofilm auf dem Prüfkörper mit einer wässrigen Lösung eines Farbstoffs an, der geeignet ist, Saccharide und/oder Proteine anzufärben. Beispiele hierfür sind: Safranin O, Coomassie Brillant Blue, Kristallviolett, Rutheniumrot und Erythrosin. Beispielsweise färbt man den Biofilm mit einer wässrigen Lösung von Safranin O ein. Die Farbstoff-Konzentration liegt vorzugsweise im Bereich von 0,001 und 0,1 Gew.-%, insbesondere um 0,01 Gew.-%. Die Färbedauer kann beispielsweise 15 Minuten betragen.

Danach spült man im Teilschritt c) den Prüfkörper mit einer zweiten wässrigen Lösung ab, die vorzugsweise ebenfalls mindestens eine Substanz enthält, die das Wachstum von Bakterien hemmt. Hierfür sind wiederum Azidionen, beispielsweise in Form von Natriumazid, geeignet. Die Konzentration an Natriumazid kann wiederum etwa 0,02 Gew.-% betragen. Zur Vereinfachung können für die Teilschritte a) und c) dieselben wässrigen Lösungen verwendet werden.

Die Intensität der Färbung des Biofilms wird geprüft, nachdem man den im Biofilm gebundenen Farbstoff von dem Prüfkörper mit einem Lösungsmittel ablöst und dann durch Vergleich mit Vergleichs-Farbstofflösungen beurteilt. Für diese Vorgehensweise ist als Lösungsmittel beispielsweise Dimethylsulfoxid geeignet. Die Extraktionsdauer für den Farbstoff kann im Bereich von 30 Minuten liegen.

Zur Kontrolle der Biofilmbildung gibt man also dann, wenn die ermittelte Intensität der Färbung des Biofilms einen vorgegebenen Schwellenwert übersteigt, dem Wassersystem mindestens ein Biozid und/oder einen Biodispergator zu. Dabei können Produkte gewählt werden, die im Stand der Technik für diesen Zweck bekannt sind. Man kann das Biozid und/oder den Biodispergator einmalig oder mehrmalig stoßweise dosieren. Oder man dosiert über einen vorbestimmten Zeitraum kontinuierlich. Die jeweils dosierten Mengen hängen von den Bedingungen des jeweiligen Wassersystems ab und werden am besten in Versuchen empirisch ermittelt. Hierfür kann man beispielsweise das erfindungsgemäße Verfahren mehrmals hintereinander mit unterschiedlicher Dosierung von Biozid und/oder Biodispergator durchführen und überprüfen, welche Mindestdosierung erforderlich ist, um die Biofilmbildung unter dem tolerierbaren Schwellenwert zu halten.

Man kann das erfindungsgemäße Verfahren auch in der Art einsetzen, daß man eine Grunddosierung von Biozid und/oder Biodispergator durchführt und durch das erfindungsgemäße Verfahren überprüft, ob diese Grunddosierung fortgesetzt, erhöht oder verringert werden sollte. Hierdurch lassen sich die Einsatzmengen von Biozid und/oder Biodispergator auf das jeweilige Wassersystem optimal anpassen.

Die Erfindung umfasst weiterhin einen Reagenziensatz, der in dem erfindungsgemäßen Verfahren verwendet werden kann. Er ist so aufgebaut, daß mit seiner Hilfe auch wenig geschulte Bearbeiter das erfindungsgemäße Verfahren durchführen können. Dieser Reagenziensatz umfasst 2 oder 3 Gefäße, in denen die in den Teilschritten a), b) und c) zu verwendenden Lösungen in anwendungsfertigen Konzentrationen vorliegen. Setzt man für die Teilschritte a) und c) dieselben wässrigen Lösungen ein, genügen 2 Gefäße. Ansonsten werden 3 benötigt. Vorzugsweise sind diese Gefäße so ausgebildet, daß ihnen auf einfache Weise direkt auch die erforderlichen Mengen der jeweiligen Lösungen entnommen werden können. Im einfachsten Fall enthalten die Gefäße jeweils diejenigen Lösungsvolumina, die in dem jeweiligen Teilschritt a), b) oder c) zu verwenden sind. In dieser Ausführungsform können die entnommenen Prüfkörper nacheinander in die Gefäße eingebracht und für die jeweiligen angegebenen Zeiträume darin gehalten werden, ohne daß Lösungen hergestellt oder abgemessen werden müssen. Alternativ hierzu und nur wenig komplizierter weisen die Gefäße jeweils Markierungen für diejenigen Lösungsvolumina auf, die in den jeweiligen Teilschritten a), b) oder c) zu verwenden sind. Man gießt dann die entsprechenden Lösungsvolumina in bereitgestellte leere Gefäße und bringt die Testkörper nacheinander in diese ein. In einer dritten Ausführungsform stellt man die 2 oder 3 Lösungen in beliebigen Volumina in beliebigen Gefäßen zur Verfügung und ergänzt dies durch 2 oder 3 leere Prüfgefäße, die jeweils eine Markierung für das erforderliche Lösungsvolumen aufweisen. Man füllt dann aus dem jeweiligen Vorratsgefäß die Lösung in das zugehörige Messgefäß bis zu dessen Markierung ein.

Für eine visuelle Auswertung muß selbstverständlich eine entsprechende Vergleichsskala wie vorstehend beschrieben oder ein Satz von Vergleichslösungen oder eine bildliche Farbdarstellung dieser Vergleichslösungen zur Verfügung gestellt werden. Diese können jedoch unabhängig von dem Reagenziensatz bereitgestellt werden.

### Ausführungsbeispiele

Nach der Entnahme der Prüfkörper aus dem Wassersystem werden folgende Schritte nacheinander ausgeführt:
1.) Fakultativ: Waschen 1 - 2 Minuten in **Lösung L1** (0,21 g/l KH₂PO₄, 9,00 g/l NaCl, 0,726 g/l Na₂HPO₄ x 7H₂O, 0,02 % NaN₃; pH = 7,4)
2.) Färbung für 15 Minuten mit **Lösung L2** (0,01 % Safranin O in Wasser)
3.) Erneutes Waschen 1 - 2 Minuten in **Lösung L3** (= H₂O; 0,02 % NaN₃)
4.) Lufttrocknung, gefolgt von optischer Auswertung anhand einer Farbintensitätsskala
5.) Alternativ: Extraktion des gebundenen Farbstoffs durch **Lösung L4** (DMSO = Dimethylsulfoxid) für 30 Minuten und Auswertung anhand einer Farbintensitätsskala.

Die Inkubationen (d.h. der Kontakt der entnommenen Prüfkörper mit den Testlösungen) können bei Raumtemperatur erfolgen. Bei einer Coupongröße von 75 x 9 mm können die Inkubationen in jeweils 12 ml der Lösungen L1, L2, L3 und L4 in verschraubbaren 15 ml-Kunststoffgefäßen durchgeführt werden. Alternativ können die erforderlichen Mengen aber auch je nach Coupongröße bereits portioniert in den entsprechenden Gefäßen mitgeliefert werden.

### Beispiel 1: Aufstellung der Korrelation von Anfärbung zu Biofilmmenge (optische vs. gravimetrische Auswertung)

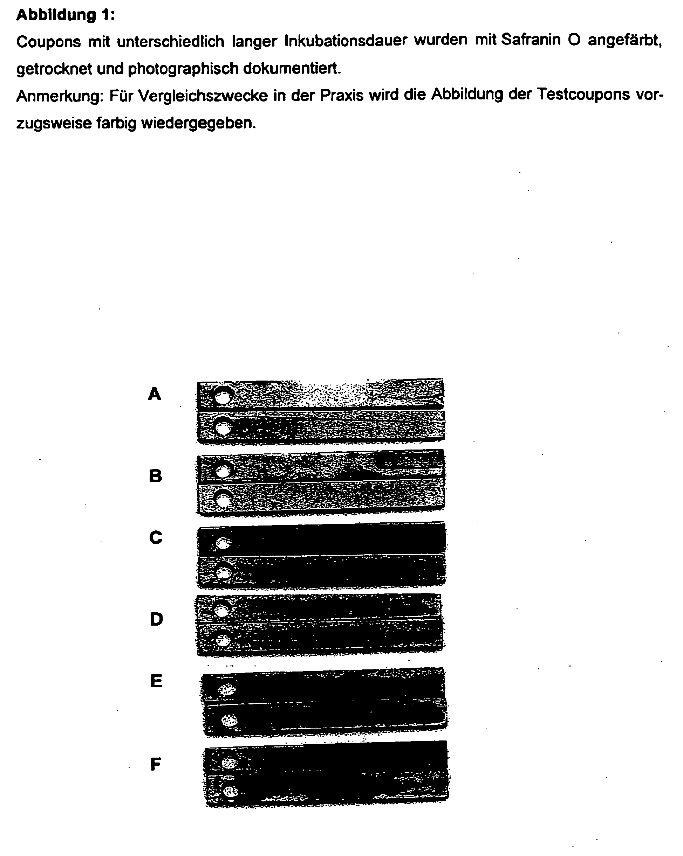

50 ml Nährmedium (Caso-Bouillon) wurden mit einer Einzelkolonie *P. aeruginosa* beimpft und für 16 Stunden bei 37 °C, 150 rpm inkubiert (Vorkultur). Biofilmcoupons (Abmessungen 75 x 9,5 x 2,5 mm, 1 Bohrung 5 mm) wurden auf der Analysenwaage gewogen, bei 160 °C 3 Stunden sterilisiert und in Einmal-Petrischalen überführt. Jede Petrischale wurde mit 30 ml Caso-Boullion, angeimpft mit der *P. aeruginosa* Vorkultur (1:100), gefüllt. Die Petrischale für den 0-Wert wurde nur mit Caso-Boullion gefüllt. Zu definierten Zeitpunkten (0, 1, 2, 2.5, 4 h) wurden je 2 Biofilmcoupons entnommen und auf Zellstoff getrocknet. Anschließend wurden die Coupons erneut gewogen und in 15 ml Falcon Tubes (Conical Centrifuge Tube Cat# 352097) überführt, in denen der Biofilm für 15 Minuten mit 12 ml der Lösung 2 (0,01 % Safranin O in H₂O) gefärbt wurde. Die Coupons wurden für 2 Minuten in Lösung 3 (H₂O; 0,02 % NaN₃) gewaschen und danach getrocknet. Die Coupons wurden mittels Scanner photographisch erfasst. Hieraus lässt sich eine bildliche Wiedergabe für Vergleichszwecke erhalten, vergl. Abb. 1.

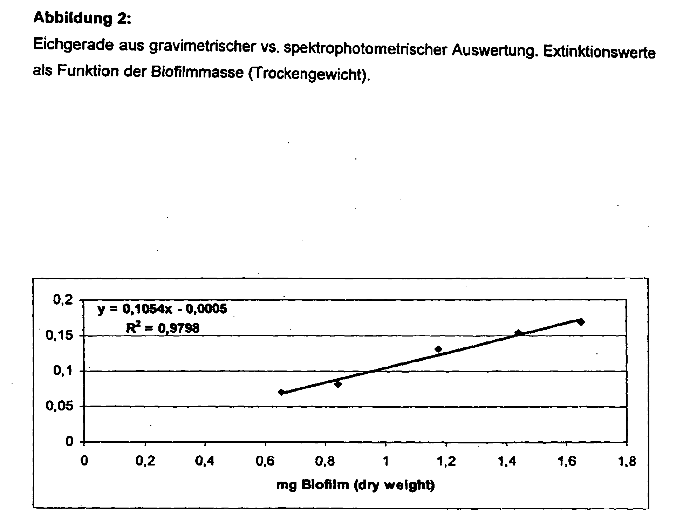

Zur Extraktion wurden die Coupons in 15 ml Falcon Tubes überführt und 12 ml Lösung 4 (DMSO) für 30 Minuten zugegeben. 200 µl der Lösungen wurden in eine 96 Well Mikrotiterplatte (Greiner) überführt. Die Extinktion wurde bei 492 nm mit einem Mikrotiterplattenreader (Tecan Spectrafluor Plus) bestimmt (Werte siehe Tabelle 1). Trägt man die Messwerte der Wägungsdifferenz und der gemessenen Extinktion gegeneinander auf, ergibt sich eine Eichgerade mit der Geradengleichung y = 0,1054x - 0,0005 und einer guten Korrelation (R² = 0,9798), die über die Geradengleichung zur Bestimmung der Biomasse mittels Färbung verwendet werden kann, vgl. Abb. 2.

**Tabelle 1: Gravimetrische und spektrophotometrische Ergebnisse von Biofilmcoupons mit unterschiedlich langer Aufwachszeit**

| | Aufwachszeit (Stunden) | Mittelwert Biofilm (mg) | Extinktion |
|---|---|---|---|
| A | 0 | 0,655 | 0,06965 |
| B | 1 | 0,845 | 0,08155 |
| C | 2 | 1,175 | 0,13125 |
| D | 2,5 | 1,44 | 0,15455 |
| E | 4 | 1,65 | 0,1679 |

### Beispiel 2:

### Praxismessung von Coupons

Je 3 Biofilm-Coupons wurden für 14 Tage mittels eines Bypasses in zwei verschiedene Kühlkreislaufsysteme (Kreislauf 1, Kreislauf 2) eingebracht. Nach dem Ausbau wurden die Coupons getrocknet und anschließend für 15 Minuten in 12 ml der Lösung 2 (0,01 % Safranin O in H₂O) in einem 15 ml Falcongefäß gefärbt. Nach Spülen in 12 ml Lösung 3 (H₂O; 0,02 % NaN₃) für 2 Minuten wurden die Coupons erneut getrocknet. Der Farbstoff wurde mit 12 ml Lösung 4 (DMSO) für 30 Minuten in einem 15 ml Falcongefäß extrahiert. 200 µl der Lösungen wurden in eine 96 Weil Mikrotiterplatte (Greiner) überführt. Die Extinktion wurde bei 492 nm am Tecan Spectrafluor Plus bestimmt.

**Tabelle 2: Biofilmbildung auf Edelstahlcoupons durch Messung der Extinktion bei 492 nm und Berechnung mittels Geradengleichung (y = 0,1054x - 0,0005)**

| | Kreislauf 1 | Kreislauf 2 |
|---|---|---|
| Coupon 1 | 0,2216 | 0,2654 |
| Coupon 2 | 0,1777 | 0,2714 |
| Coupon 3 | 0,2203 | 0,2238 |
| Mittelwert | 0,2065 | 0,2535 |
| mg Biofilm (Trockengewicht) | 1,96 | 2,41 |

### Abbildung 1:

Coupons mit unterschiedlich langer Inkubationsdauer wurden mit Safranin O angefärbt, getrocknet und photographisch dokumentiert.

Anmerkung: Für Vergleichszwecke in der Praxis wird die Abbildung der Testcoupons vorzugsweise farbig wiedergegeben.

### Abbildung 2:

Eichgerade aus gravimetrischer vs. spektrophotometrischer Auswertung. Extinktionswerte als Funktion der Biofilmmasse (Trockengewicht).

## Patentansprüche

1. Verfahren zur Kontrolle der Biofilmbildung in einem Wassersystem, wobei man in das Wassersystem selbst oder in eine Zweigleitung einen oder mehrere Prüfkörper einbringt und nach einer vorgewählten Zeitdauer entnimmt, **dadurch gekennzeichnet, dass** man
a) fakultativ den Prüfkörper mit einer ersten wässrigen Lösung abspült,
b) anschließend den Biofilm auf dem Prüfkörper mit einer Farbstofflösung einfärbt,
c) anschließend den Prüfkörper mit einer zweiten wässrigen Lösung abspült, deren Zusammensetzung der ersten wässrigen Lösung entsprechen oder von dieser verschieden sein kann, und anschließend die Intensität der Färbung des Biofilms ermittelt, indem man
d) den im Biofilm gebundenen Farbstoff von dem Prüfkörper mit einem Lösungsmittel ablöst, wobei man eine Farbstofflösung erhält, deren Farbintensität man durch Vergleich mit Vergleichs-Farbstofflösungen beurteilt,
e) und dann, wenn die im Teilschritt d) ermittelte Intensität der Färbung des Biofilms einen vorgegebenen Schwellenwert übersteigt, dem Wassersystem mindestens ein Biozid und/oder einen Biodispergator zugibt.

2. Verfahren nach Anspruch 1, **dadurch gekennzeichnet, dass** man die flächenbezogene Trockenmasse des Biofilms auf dem Prüfkörper dadurch feststellt, dass man die im Teilschritt d) ermittelte Intensität der Färbung des Biofilms mit der auf dieselbe Weise ermittelten Intensität der Färbung von Biofilmen mit bekannten flächenbezogenen Trockenmassen vergleicht.

3. Verfahren nach einem oder mehreren der Ansprüche 1 bis 2, **dadurch gekennzeichnet, dass** der Prüfkörper flächenförmig ausgebildet ist.

4. Verfahren nach einem oder mehreren der Ansprüche 1 bis 3, **dadurch gekennzeichnet, dass** man den Prüfkörper nach einer vorgewählten Zeitdauer im Bereich von fünf Tagen und fünf Monaten, vorzugsweise im Bereich von zwei Wochen bis 2 Monaten entnimmt.

5. Verfahren nach einem oder mehreren der Ansprüche 1 bis 4, **dadurch gekennzeichnet, dass** man den Prüfkörper so in das Wassersystem oder eine Zweigleitung einbringt, dass nach dem Einbringen kein Licht auf den Prüfkörper fällt.

6. Verfahren nach einem oder mehreren der Ansprüche 1 bis 5, **dadurch gekennzeichnet, dass** man den Prüfkörper im Teilschritt a) mit einer wässrigen Lösung abspült, die auf einen pH-Wert im Bereich von 7,0 bis 8,0 abgepuffert ist und die mindestens eine Substanz enthält, die das Wachstum von Bakterien hemmt, vorzugsweise Azidionen.

7. Verfahren nach einem oder mehreren der Ansprüche 1 bis 6, **dadurch gekennzeichnet, dass** man im Teilschritt b) den Biofilm auf dem Prüfkörper mit einer wässrigen Lösung eines Farbstoffs einfärbt, der ausgewählt ist aus Safranin O, Coomassie Brilliant Blue, Kristallviolett, Rutheniumrot und Erythrosin.

8. Verfahren nach einem oder mehreren der Ansprüche 1 bis 7, **dadurch gekennzeichnet, dass** man den Prüfkörper im Teilschritt c) mit einer wässrigen Lösung abspült, die mindestens eine Substanz enthält, die das Wachstum von Bakterien hemmt, vorzugsweise Azidionen.

## Claims

1. Process for controlling the formation of biofilm in a water system, where one or several test specimens are brought into the water system itself or into a branch line and removed after a preselected period of time, **characterised by** the fact that
a) the test specimen is optionally rinsed off with an aqueous solution,
b) subsequently, the biofilm on the test specimen is coloured with a dye solution, and
c) subsequently, the test specimen is rinsed off with a second aqueous solution whose composition can correspond to the first aqueous solution or may be different from it, and subsequently the intensity of the colouring of the biofilm is ascertained
d) by stripping the dye bound in the biofilm from the test specimen with a solvent where a dye solution is obtained whose colour intensity is assessed by comparing it with comparable dye solutions, and
e) then, if the intensity of the colouring of the biofilm ascertained in the individual step is in excess of a specified threshold value, at least one biocide and/or one biodispergator is added to the water system.

2. Process in accordance with claim 1, **characterised by** the fact that the area-related dry mass of the biofilm on the test specimen is established by comparing the intensity of the colouring of the biofilm ascertained in the individual step with the intensity of the colouring of the biofilms ascertained in the same fashion with known area-related dry masses.

3. Process in accordance with one or several of the claims from 1 through to 2, **characterised by** the fact that the test specimen has formed a planiform.

4. Process in accordance with one or several of the claims from 1 through to 3, **characterised by** the fact that the test specimen is removed after a preselected period of time in the range of five days and five months, preferably in the range of two weeks to 2 months.

5. Process in accordance with one or several of the claims from 1 through to 4, **characterised by** the fact that the test specimen is brought into the water system or a branch line in such a fashion that no light falls on the test specimen after being brought in.

6. Process in accordance with one or several of the claims from 1 through to 5, **characterised by** the fact that the test specimen is rinsed off with an aqueous solution in an individual step a) that is buffered off to a pH value in the range of 7.0 to 8.0 and that contains at least one substance that retards the growth of bacteria, preferably acidiones.

7. Process in accordance with one or several of the claims from 1 through to 6, **characterised by** the fact that the biofilm on the test specimen is coloured in an individual step b) with an aqueous solution of a dye that is selected from saffranine O, coomassie brilliant blue, crystal violet, ruthenium red and erythrosin.

8. Process in accordance with one or several of the claims from 1 through to 7, **characterised by** the fact that the test specimen is rinsed off in an individual step c) with an aqueous solution that contains at least one substance that retards the growth of bacteria, preferably acidiones.

## Revendications

1. Procédé de contrôle de la formation de biofilms dans un système aqueux, par lequel l'on intègre dans le système aqueux lui-même ou dans une dérivation un ou plusieurs blocs d'essai retirés après une durée prédéterminée, **caractérisé en ce que** l'on
a) rince facultativement le bloc d'essai avec une première solution aqueuse,
b) teint ensuite le biofilm sur le bloc d'essai avec une solution de colorant,
c) rince ensuite le bloc d'essai avec une seconde solution aqueuse dont la composition peut être identique à, ou différer de, la première solution, puis l'on calcule l'intensité de la coloration du film en
d) détachant le colorant attaché dans le biofilm du bloc d'essai à l'aide d'un solvant afin d'obtenir une solution de colorant dont l'intensité est jugée en la comparant avec des solutions de colorants comparatives,
e) et enfin, une fois que l'intensité de la coloration du biofilm calculée à la sous-étape d) dépasse une valeur seuil prédéfinie, l'on ajoute au système aqueux au moins un biocide et/ou un biodispersant.

2. Procédé selon la revendication 1, **caractérisé en ce que** l'on identifie la matière sèche relative à la surface du biofilm sur le bloc d'essai en comparant l'intensité de la coloration du biofilm, calculée à la sous-étape d), avec l'intensité, calculée de la même manière, de la coloration de biofilms présentant des matières sèches relatives à des surfaces connues.

3. Procédé selon l'une ou plusieurs des revendications 1 à 2, **caractérisé en ce que** le bloc d'essai est réalisé de forme plane.

4. Procédé selon l'une ou plusieurs des revendications 1 à 3, **caractérisé en ce que** l'on retire le bloc d'essai après une durée prédéterminée allant de 5 jours à 5 mois, de préférence de 2 semaines à 2 mois.

5. Procédé selon l'une ou plusieurs des revendications 1 à 4, **caractérisé en ce que** l'on intègre le bloc d'essai dans le système aqueux ou dans une dérivation de sorte qu'aucune lumière ne baigne le bloc d'essai une fois celui-ci intégré.

6. Procédé selon l'une ou plusieurs des revendications 1 à 5, **caractérisé en ce que**, à la sous-étape a), l'on rince le bloc d'essai avec une solution aqueuse atténuée à un pH entre 7,0 et 8,0 et qui contient au moins une substance inhibant la croissance de bactéries, de préférences des ions acides.

7. Procédé selon l'une ou plusieurs des revendications 1 à 6, **caractérisé en ce que**, à la sous-étape b), l'on colore le biofilm sur le bloc d'essai à l'aide d'une solution aqueuse d'un colorant choisi parmi la safranine O, le bleu brillant de Coomassie, le violet cristallisé, le rouge de ruthénium et l'érythrosine.

8. Procédé selon l'une ou plusieurs des revendications 1 à 7, **caractérisé en ce que**, à la sous-étape c), l'on rince le bloc d'essai avec une solution aqueuse contenant au moins une substance inhibant la croissance de bactéries, de préférences des ions acides.
